# EUROPÄISCHE PATENTSCHRIFT

(11) **EP 3 394 611 B1**
(45) Veröffentlichungstag und Bekanntmachung des Hinweises auf die Patenterteilung: **17.02.2021**
(21) Anmeldenummer: 16822668.6
(22) Anmeldetag: 20.12.2016
(51) Int. Cl.: G01N 33/24, G01J 3/443, G01N 21/71

(54) **VERFAHREN ZUR ANALYSE EINES PROBENMATERIALS**
METHOD FOR ANALYZING A SAMPLE MATERIAL
PROCÉDÉ D'ANALYSE D'UN MATÉRIAU ÉCHANTILLON

(30) Priorität: 21.12.2015 DE 102015122408
(43) Veröffentlichungstag der Anmeldung: 31.10.2018
(73) Patentinhaber: thyssenkrupp Industrial Solutions AG, 45143 Essen (DE); thyssenkrupp AG, 45143 Essen (DE)
(72) Erfinder: TEUTENBERG, Reinhard, 59423 Unna (DE); SCHNEBERGER, Jürgen, 59320 Ennigerloh (DE); BORNEFELD, Marc, 33617 Bielefeld (DE); MAIER, Oliver, 48167 Münster (DE)
(74) Vertreter: thyssenkrupp Intellectual Property GmbH
(86) Internationale Anmeldenummer: PCT/EP2016/081951
(87) Internationale Veröffentlichungsnummer: WO 2017/108810

(56) Entgegenhaltungen:
- US-A- 3 890 089
- XU TAO ET AL: "Multi-elemental surface mapping and analysis of carbonaceous shale by laser-induced breakdown spectroscopy", SPECTROCHIMICA ACTA. PART B: ATOMIC SPECTROSCOPY, Bd. 115, 28. Oktober 2015 (2015-10-28), Seiten 31-39, XP029367099, ISSN: 0584-8547, DOI: 10.1016/J.SAB.2015.10.008
- C. ARAGÓN ET AL: "Measurement of Stark broadening parameters of Fe II and Ni II spectral lines by laser induced breakdown spectroscopy using fused glass samples", JOURNAL OF QUANTITATIVE SPECTROSCOPY AND RADIATIVE TRANSFER, Bd. 134, Oktober 2015 (2015-10), Seiten 39-45, XP055348200, GB ISSN: 0022-4073, DOI: 10.1016/j.jqsrt.2013.10.011
- ALICE STANKOVA ET AL: "Comparison of LA-ICP-MS and LA-ICP-OES for the analysis of some elements in fly ashes", JOURNAL OF ANALYTICAL ATOMIC SPECTROMETRY, Bd. 26, Nr. 2, Januar 2011 (2011-01), Seiten 443-449, XP055348546, ISSN: 0267-9477, DOI: 10.1039/C0JA00020E
- V. MOTTO-ROS ET AL: "Precise alignment of the collection fiber assisted by real-time plasma imaging in laser-induced breakdown spectroscopy", SPECTROCHIMICA ACTA PART B: ATOMIC SPECTROSCOPY, Bd. 92, Februar 2014 (2014-02), Seiten 60-69, XP055183416, ISSN: 0584-8547, DOI: 10.1016/j.sab.2013.12.008
- JULIEN MALHERBE ET AL: "Elemental analyses of soil and sediment fused with lithium borate using isotope dilution laser ablation-inductively coupled plasma-mass spectrometry", ANALYTICA CHIMICA ACTA, Bd. 793, September 2013 (2013-09), Seiten 72-78, XP055348242, AMSTERDAM, NL ISSN: 0003-2670, DOI: 10.1016/j.aca.2013.07.031

## Beschreibung

Die Erfindung betrifft ein Verfahren zur Analyse eines Probenmaterials, bei dem das Probenmaterial gemahlen, in eine Tablettenform überführt, gegebenenfalls weiter bearbeitet und anschließend analysiert wird. Die Erfindung betrifft weiterhin eine zur Durchführung eines solchen Verfahrens geeignete Anlage.

Es ist bekannt, derartige Tabletten als Presstabletten herzustellen, indem das Probenmaterial gemahlen und unter Anwendung von Druck und/oder Bindemittel zur Tablette weiterverarbeitet wird.

Es ist ferner bekannt, analysenfertige Tabletten aus einer Schmelze herzustellen. Dabei wird das Probenmaterial mit einem Schmelzmittel vermischt, diese Probenmaterial-Schmelzmittel-Mischung aufgeschmolzen und die Schmelze in eine Tablettenform gegossen und darin abgekühlt. Ein solches Abkühlen unter gleichzeitiger Formgebung ist jedoch verfahrenstechnisch sowie in apparativer Hinsicht aufwändig. So muss die Abkühlung einer Schmelztablette unter sehr kontrollierten Bedingungen erfolgen, da ein zu rasches Abkühlen zum Brechen der Tablette führen kann, während bei einem zu langsamen Abkühlen die Schmelze kristallisieren kann, wodurch die Tablette u.a. ihre Festigkeit verlieren würde.

Aus der WO 2015/000571 A1 und aus der US 5,257,302 sind jeweils Verfahren zur Herstellung einer ein Probenmaterial umfassenden Tablette bekannt, bei denen eine das Probenmaterial umfassende Materialmischung aufgeschmolzen und wieder abgekühlt wird. Das so entstandene glasige Material wird anschließend gemahlen und daraufhin zu der Tablette verpresst.

In der Veröffentlichung "Multi-elemental surface mapping and analysis of carbonaceous shale by Iaser-induced breakdown spectroscopy" von Tao Xu et al., aus Spectrochimica Acta Part B 115 (2016), Seiten 31 bis 39, ist ein Verfahren zur Vorbereitung einer Tablette für eine LIBS-Analyse offenbart, bei dem ein Probenmaterial gemahlen und anschließend zu einer Tablette gepresst wird.

Auch in der Veröffentlichung "Measurement of Stark broadening parameters of Fe II and Ni II spectral lines by laser induced breakdown spectroscopy using fused glass samples" von C. Aragón et al., aus Journal of Quantitative Spectroscopy & Radiative Transfer 134 (2014), Seiten 39 bis 45, ist ein Verfahren zur Vorbereitung einer Tablette für eine LIBS-Analyse offenbart, bei dem ein pulverförmiges Probenmaterial in einem Schmelztiegel zu einer Tablette geformt wird. Weiterhin ist darin ein Mischen des Probenmaterials mit Schmelzmittel (u.a. Lithiumtetraborat) beschrieben.

In der Veröffentlichung "Comparison of LA-ICP-MS and LA-ICP-OES for the analysis of some elements in fly ashes" von Alice Stankova et al., aus Journal of Analytical Atomic Spectrometry, 2011, 26, Seiten 443 bis 449, ist ein Verfahren zur Vorbereitung einer Tablette für eine LA-ICP-MS-Analyse, bei dem ein pulverförmiges Probenmaterial in einem Schmelztiegel zu einer Tablette geformt wird, offenbart. Weiterhin ist auch in dieser Veröffentlichung ein Mischen des Probenmaterials mit Schmelzmittel (Lithiumtetraborat) beschrieben.

Die Veröffentlichung "Precise alignment of the collection fiber assisted by real-time plasma imaging in laser-induced breakdown spectroscopy") von V. Motto-Ros et al. aus Spectrochimica Acta Part B 92 (2014), Seiten 60 bis 69, beschreibt die Kalibrierung einer Vorrichtung zur Analyse von Probenmaterial mittels LIBS, wobei glasige Referenzproben mit Lithiumborat verwendet werden, die mittels der "fused bead"-Technologie hergestellt worden sind.

In der Veröffentlichung "Elemental analyses of soil and sediment fused with lithium borate using isotope dilution laser ablation-inductively coupled plasma-mass spectrometry" von Julien Malherbe et al., aus Analytica Chimica Acta 793 (2013), Seiten 72 bis 78, ist ein Verfahren zur Vorbereitung einer Tablette für eine LA-ICP-MS-Analyse, bei dem eine pulverförmige Mischung aus Probenmaterial und Schmelzmittel (Lithiumborat) in einem Schmelztiegel zu einer Tablette geformt wird, beschrieben. Die Probenmaterial-Schmelzmittel-Mischung wird dabei mehrstufig erhitzt.

Die US 3,890,089 A offenbart ein Verfahren sowie eine Anlage zur Herstellung einer ein Probenmaterial umfassenden Schmelztablette für eine spätere Analyse, insbesondere Röntgenfluoreszenzanalyse.

Der Erfindung lag die Aufgabe zugrunde, ein verbessertes Verfahren zur Analyse eines Probenmaterials anzugeben.

Diese Aufgabe wird mittels eines Verfahrens gemäß dem Patentanspruch 1 gelöst. Vorteilhafte Ausführungsformen des erfindungsgemäßen Verfahrens sind Gegenstände der weiteren Patentansprüche und ergeben sich aus der nachfolgenden Beschreibung der Erfindung.

Bei einem Verfahren zur Analyse eines Probenmaterials, bei dem das Probenmaterial gemahlen, zu einer Tablette geformt und anschließend analysiert wird, ist zum einen vorgesehen, dass die Analyse mittels Laseremissionsspektroskopie durchgeführt wird. Weiterhin ist vorgesehen, dass das Probenmaterial (vorzugsweise vor dem Formen der Tablette) zumindest teilweise mit einem Schmelzmittel (insbesondere ein Schmelzsalz und/oder eine Säure) vermischt wird. Durch eine Verdünnung der Probe mit einem Schmelzmittel kann die Probenmatrix vereinheitlicht und dadurch der sogenannte Matrixeffekt im Rahmen der Analyse verringert werden. Weiterhin ist im Rahmen eines erfindungsgemäßen Verfahrens vorgesehen, dass das Probenmaterial in einem ersten Mahlschritt vorgemahlen und nach dem Vermischen mit dem Schmelzmittel in einem weiteren Mahlschritt weitergemahlen wird. In diesem Fall kann das Mahlen der Probenmaterial-Schmelzmittel-Mischung primär dem Zweck dienen, das bereits gemahlene Probenmaterial mit dem Schmelzmittel zu vermischen. Dies wird insbesondere dadurch ermöglicht, dass Schmelzmittel vielfach bereits in feinkörniger Form vorliegen. Ein Vorteil, der sich daraus ergeben kann, liegt darin, dass sowohl das Mahlen des Probenmaterials als auch das Vermischen des Probenmaterials mit dem Schmelzmittel in derselben Vorrichtung, nämlich einer geeigneten (Fein)Mühle, durchgeführt werden kann. Ein Bereitstellen einer zusätzlichen Mischvorrichtung kann dadurch vermieden werden.

Dementsprechend wird unter "Mahlen" erfindungsgemäß nicht zwingend ein Verfahrensschritt verstanden, bei dem eine Bearbeitung eines Materials oder Materialgemisches mit einem Erreichen einer Verkleinerung der Korngröße des Materials oder Materialgemisches verknüpft ist. Vielmehr kann ein solches "Mahlen" ausschließlich oder primär einer Durchmischung eines Materialgemisches dienen, sofern durch den gleichen Verfahrensablauf beziehungsweise durch die Verwendung derselben dabei genutzten Vorrichtung (Mühle) grundsätzlich, d.h. bei sich davon unterscheidenden Verfahrensparametern, ein Mahlen des Materials beziehungsweise Materialgemisches möglich wäre. Die Drehzahl der (Fein-)Mühle kann an den zu erzielenden Mischeffekt angepasst werden (ggf. geringe Geschwindigkeit) und kann sich von einer Mahldrehzahl unterscheiden.

Eine gute Durchmischung des Schmelzmittels mit dem Probenmaterial kann bei einem anschließenden Aufschmelzen, insbesondere bei einem Aufschmelzen mittels Laserstrahlung, den Vorteil aufweisen, dass dann jede sogenannte "Pfütze" (Zone, die umgeschmolzen wird) möglichst gleichmäßig zusammengesetzt ist (Korngrößenverteilung von Probe zu Flussmittel immer möglichst gleich).

Gegebenenfalls kann auch vorgesehen sein, das Schmelzmittel dosiert in Form eines Festkörpers mit dem Probenmaterial zu vermischen, wodurch eine Dosierung von Probenmaterial und Schmelzmittel in einem vordefinierten Verhältnis vereinfacht werden kann. In diesem Fall kann es sinnvoll sein, den Schmelzmittel-Festkörper in zumindest einem der Mahlschritte auch zu mahlen d.h. zu Partikeln definierter (maximaler) Korngröße zu zerkleinern.

Eine zur Durchführung eines erfindungsgemäßen Verfahrens geeignete Anlage umfasst zumindest eine Mühle zum Mahlen des Probenmaterials und eine Vorrichtung zum Formen einer Tablette aus dem gemahlenen Probenmaterial (vorzugsweise eine Tablettenpresse). Weiterhin umfasst eine solche Anlage eine Vorrichtung zur Analyse des Probenmaterials mittels Laseremissionsspektroskopie sowie eine Mischvorrichtung zum Mischen des Probenmaterials mit einem Schmelzmittel.

Bei der Laseremissionsspektroskopie, die auch als laserinduzierte Plasmaspektroskopie oder mit der Kurzbezeichnung LIBS, die sich aus dem englischen Ausdruck "laser-induced breakdown spectroscopy" ableitet, bezeichnet wird, handelt es sich um ein Verfahren zur Bestimmung der chemischen Zusammensetzung eines Probenmaterials, bei dem das Probenmaterial mit relativ kurzen Laserimpulsen beaufschlagt wird, wodurch relativ kleine Mengen des Probenmaterials verdampft und ionisiert werden. Beim darauffolgenden Zerfall des Plasmas wird Licht emittiert, das charakteristisch für die in dem Probenmaterial enthaltenen chemischen Elemente ist. Das Spektrum des Lichts kann dann mittels eines Spektrometers ausgewertet werden.

Ein Vorteil der Laseremissionsspektroskopie ist die relativ einfache Durchführbarkeit sowie die Tatsache, dass für diese keine besonders hohen Anforderungen an eine Aufbereitung des Probenmaterials gestellt sind. Durch eine Anwendung bei Probenmaterial, das in einer Tablettenform mit homogener, repräsentativer Oberfläche vorliegt, kann für die Laseremissionsspektroskopie auf einfache Weise eine hohe Analysegenauigkeit realisiert werden.

Als "Tablette" wird erfindungsgemäß ein Festkörper in einer definierten räumlichen Form verstanden, der zumindest teilweise aus dem Probenmaterial oder einem das Probenmaterial umfassenden Materialgemisch besteht.

Weiterhin bevorzugt kann vorgesehen sein, dass im Rahmen der Durchführung der Laseremissionsspektroskopie zumindest eine Oberfläche der Tablette an mehreren Stellen und insbesondere auch nur punktuell (d.h. ausschließlich in einer Fläche, die der Wirkungsfläche des Lasers entspricht) mittels des hierfür eingesetzten Lasers beaufschlagt wird. Dies ermöglicht, eine Analyse an verschiedenen Teilmengen des Probenmaterials durchzuführen und die so erhaltenen Einzelanalyseergebnisse zu einem Gesamtanalyseergebnis zusammenzuführen insbesondere zu mitteln, wodurch sich eine höhere Genauigkeit für das als Endergebnis genutzte Gesamtanalyseergebnis ergeben kann.

In einer vorteilhaften Ausführungsform des erfindungsgemäßen Verfahrens kann zudem vorgesehen sein, dass das Probenmaterial oder die Probenmaterial-Schmelzmittel-Mischung zumindest teilweise vor der Analyse aufgeschmolzen wird. Eine Analyse mittels Laseremissionsspektroskopie kann dann unmittelbar an dem aufgeschmolzenen Probenmaterial oder der aufgeschmolzenen Probenmaterial-Schmelzmittel-Mischung, bei einem vorzugsweise vorgesehenen lokal begrenzten und insbesondere punktuellen Aufschmelzen beispielsweise an den dabei entstehenden Schmelzpfützen, durch geführt werden.

Eine erfindungsgemäße Anlage kann hierfür eine entsprechende Vorrichtung zum Aufschmelzen des Probenmaterials (bzw. einer das Probenmaterial umfassenden Materialmischung) aufweisen.

Durch das Aufschmelzen des Probenmaterials oder der Probenmaterial-Schmelzmittel-Mischung kann erreicht werden, dass diese in eine homogene beziehungsweise chemisch homogene Schmelze überführt wird, was eine Analyse des Probenmaterials anhand der Schmelze zu verbessern hilft. Dadurch können Einflüsse, die sich beispielsweise aus der Korngrößenverteilung und der Dichte sowie aus mineralogischen Eigenschaften, wie beispielsweise Kristallstruktur und Kristallinität, der Originalprobe ergeben, vermindert oder ganz beseitigt werden. In Abhängigkeit von dem verwendeten Probenmaterial (bzw. der das Probenmaterial umfassenden Materialmischung) kann durch das Aufschmelzen auch Eisen an Bor angebunden werden, wodurch sich Vorteile bei der Kalibration ergeben können.

In einer alternativen Ausführungsform eines erfindungsgemäßen Verfahrens kann vorgesehen sein, dass die Tablette ohne ein vorhergehendes Aufschmelzen des Probenmaterials (bzw. einer das Probenmaterial umfassenden Materialmischung) mittels Laseremissionsspektroskopie analysiert wird.

In einer bevorzugten Weiterbildung eines erfindungsgemäßen Verfahrens, bei dem das Probenmaterial oder die Probenmaterial-Schmelzmittel-Mischung zumindest teilweise vor der Analyse aufgeschmolzen wird, kann vorgesehen sein, dass vor der Analyse eine Erstarrung der Schmelze herbeigeführt wird, wobei die erstarrte Schmelze die Tablettenform bereits aufweist oder erst anschließend in die Tablettenform überführt wird. Dadurch können Tabletten für eine spätere Analyse mittels Laseremissionsspektroskopie vorbereitet werden.

In einer weiterhin bevorzugten Weiterbildung eines erfindungsgemäßen Verfahrens, bei dem das Probenmaterial oder die Probenmaterial-Schmelzmittel-Mischung zumindest teilweise vor der Analyse aufgeschmolzen wird, kann vorgesehen sein, dass das Probenmaterial oder die Probenmaterial-Schmelzmittel-Mischung während des Aufschmelzens (aktiv) gekühlt wird. Dies kann beispielsweise mittels einer Luftströmung und insbesondere mittels Drucklufts erfolgen. Die erfindungsgemäße Anlage kann hierfür eine entsprechende Kühlvorrichtung aufweisen. Ohne eine solche Kühlung kann es (verstärkt) zu einer Rekristallisation des Probenmaterial oder der Probenmaterial-Schmelzmittel-Mischung kommen.

Alternativ oder ergänzend kann bei einem erfindungsgemäßen Verfahren, bei dem das Probenmaterial oder die Probenmaterial-Schmelzmittel-Mischung zumindest teilweise vor der Analyse aufgeschmolzen wird, auch vorgesehen sein, dass zumindest ein für ein Aufschmelzen vorgesehener Anteil des Probenmaterials oder der Probenmaterial-Schmelzmittel-Mischung vor dem Aufschmelzen vorgewärmt und/oder während des Aufschmelzens ergänzend erwärmt wird. Die erfindungsgemäße Anlage kann hierfür eine entsprechende Heizvorrichtung aufweisen. Ein Vorwärmen und/oder ergänzendes Erwärmen des Probenmaterials oder der Probenmaterial-Schmelzmittel-Mischung kann Spannungen in der Tablette verhindern oder verringern, wodurch gegebenenfalls Abplatzungen oder Risse in der Tablette während des Aufschmelzens verhindert werden können. Das Vorwärmen und/oder das ergänzende Erwärmen der Tablette kann beispielsweise mittels einer Heizplatte ("Widerstandsheizvorrichtung"), einem (Hochtemperatur-)IR-Strahler oder auch mittels Lasers erfolgen. Das Probenmaterial oder die Probenmaterial-Schmelzmittel-Mischung kann dabei von der (für das Aufschmelzen vorgesehenen) Oberseite oder der Unterseite her erwärmt werden. In dem vorzuwärmenden oder ergänzend zu erwärmenden Material können beispielsweise Temperaturen von zwischen 400°C und 600°C eingestellt werden. Ein einzustellende Temperatur oder ein einzustellender Temperaturbereich kann dabei geregelt werden.

In einer bevorzugten Weiterbildung eines erfindungsgemäßen Verfahrens, bei dem das Probenmaterial oder die Probenmaterial-Schmelzmittel-Mischung zumindest teilweise vor der Analyse aufgeschmolzen wird, kann vorgesehen sein, dass das Probenmaterial oder die Probenmaterial-Schmelzmittel-Mischung mittels Laserstrahlung, Plasmastrahlung, Hochtemperatur-Infrarotstrahlung, Mikrowellenstrahlung und/oder Elektronenstrahlung aufgeschmolzen wird. Durch eine Verwendung von Laserstrahlung, Plasmastrahlung, Hochtemperatur-Infrarotstrahlung, Mikrowellenstrahlung und/oder Elektronenstrahlung wird eine sehr exakt definierbare, lokal begrenzbare und/oder lokal sehr hohe Energieeinbringung in das aufzuschmelzende Probenmaterial oder die aufzuschmelzende Probenmaterial-Schmelzmittel-Mischung ermöglicht, was sich positiv auf das Aufschmelzen auswirken und insbesondere auch eine einfache und gleichzeitig sehr exakte Regelung des Aufschmelzprozesses ermöglichen kann. Durch eine lokal exakt definierte Energieeinbringung kann zudem die bei der Durchführung eines erfindungsgemäßen Verfahrens anfallende Verlustwärme minimiert werden. Auch kann durch die erfindungsgemäße Verwendung von Laserstrahlung, Plasmastrahlung, Hochtemperatur-Infrarotstrahlung, Mikrowellenstrahlung und/oder Elektronenstrahlung ein vergleichsweise schnelles Aufschmelzen des hierfür vorgesehenen Probenmaterials oder der hierfür vorgesehenen Probenmaterial-Schmelzmittel-Mischung erreicht werden, was sich in einem entsprechenden Maße vorteilhaft auf die für die Herstellung der Tablette benötigte Zeit auswirken kann.

Bei einem erfindungsgemäßen Verfahren, bei dem das Probenmaterial oder die Probenmaterial-Schmelzmittel-Mischung zumindest teilweise vor der Analyse aufgeschmolzen wird und zudem ein Vorwärmen zumindest eines für das Aufschmelzen vorgesehenen Anteils des Probenmaterials oder der Probenmaterial-Schmelzmittel-Mischung vorgesehen ist, kann weiterhin vorgesehen sein, dass zumindest der für das Aufschmelzen vorgesehene Anteil des Probenmaterials oder der Probenmaterial-Schmelzmittel-Mischung mittels Laserstrahlung vorgewärmt wird und das Probenmaterial oder die Probenmaterial-Schmelzmittel-Mischung mittels Laserstrahlung desselben Lasers aufgeschmolzen wird. Dadurch kann sich in vorteilhafter Weise eine Doppelnutzung eines Lasers ergeben, durch die der konstruktive Aufwand für eine erfindungsgemäße Anlage gering gehalten werden kann.

Bei einem Aufschmelzen des Probenmaterials oder der Probenmaterial-Schmelzmittel-Mischung mittels Laserstrahlung kann dies durch denselben Laser bewirkt werden, der auch im Rahmen der Analyse mittels Laseremissionsspektroskopie eingesetzt wird. Dieser Laser kann dabei auch noch einem Vorwärmen des Probenmaterials oder der Probenmaterial-Schmelzmittel-Mischung dienen. Alternativ kann für das Aufschmelzen jedoch auch ein anderer Laser genutzt werden.

Bei der Durchführung einer ein Aufschmelzen des Probenmaterials oder der Probenmaterial-Schmelzmittel-Mischung umfassenden Ausführungsform eines erfindungsgemäßen Verfahrens kann zudem noch vorgesehen sein, dass das Probenmaterial oder die Probenmaterial-Schmelzmittel-Mischung selektiv mittels der Laserstrahlung, Plasmastrahlung und/oder Elektronenstrahlung aufgeschmolzen wird. Dabei wird unter "selektiv" ein nach und nach erfolgendes Aufschmelzen der Probenmaterial-Schmelzmittel-Mischung beziehungsweise des hierfür vorgesehenen Anteils davon verstanden, indem der Laserstrahl, der Plasmastrahl und/oder der Elektronenstrahl, dessen Abdeckung kleiner als die von der aufzuschmelzenden Probenmaterial-Schmelzmittel-Mischung bedeckte Fläche ist, entlang dieser Fläche verfahren wird, so dass die Probenmaterial-Schmelzmittel-Mischung über dieser Fläche nach und nach aufgeschmolzen wird. Dabei kann beim Aufschmelzen einer Teilfläche eine zuvor bereits aufgeschmolzene andere Teilfläche bereits wieder erstarren.

Durch die Anzahl der Bestrahlungsschritte (Einfach- oder Mehrfachbestrahlung) kann der Schmelzvorgang beeinflusst werden. Bei der Einfachbestrahlung überfährt beispielsweise ein Laserstrahl (auch gepulster Laserstrahl) jeden "Punkt" exakt einmal (z.B. nur in Vorwärtsbewegung). Bei der Mehrfachbestrahlung überfährt beispielsweise der Laserstrahl jeden "Punkt" mehrmals. Der Vorwärtsbewegung wird dabei eine Rückwärts und/oder Seitwärtsbewegung überlagert. Die Art der Bestrahlung ist materialabhängig einstellbar (u.a. abhängig vom Probenmaterial).

Für eine Analyse mittels Laseremissionsspektroskopie kann vorzugsweise ein YAG-Laser zum Einsatz kommen. Dieser kann dabei weiterhin bevorzugt gepulst (z.B. Nanosekunden-Impulse) betrieben werden. Für ein vorzugsweise vorgesehenes Vorwärmen und/oder Aufschmelzen des Probenmaterials oder der Probenmaterial-Schmelzmittel-Mischung kann ebenfalls vorteilhaft ein YAG-Laser, alternativ beispielsweise aber auch ein CO₂-Laser, eingesetzt werden. Der dafür verwendete Laser kann dabei vorzugsweise kontinuierlich, d.h. nicht gepulst, betrieben werden.

Im Rahmen eines erfindungsgemäßen Verfahrens kann weiterhin bevorzugt vorgesehen sein, dass das Probenmaterial bis zum Erreichen einer Korngröße von maximal 100 µm, besonders bevorzugt von maximal 63 µm, gemahlen wird. Dies wirkt sich vorteilhaft auf die Homogenisierung des Probenmaterials oder der Probenmaterial-Schmelzmittel-Mischung im Rahmen des Aufschmelzens aus.

In einer weiterhin bevorzugten Ausführungsform des erfindungsgemäßen Verfahrens kann vorgesehen sein, dass die Erstarrung der Schmelze derart herbeigeführt wird, dass ein glasiger Bestandteil (die wieder erstarrte Schmelze) der Tablette ausgebildet wird. In bestimmten Ausführungsformen des erfindungsgemäßen Verfahrens kann aber auch eine teilkristalline Ausbildung der erstarrten Schmelze ausreichend oder sogar vorteilhaft sein.

Dabei wird unter "glasig" beziehungsweise "glasiger Bestandteil" ein amorphes Materialgefüge verstanden, das nach dem Abkühlen aus der Schmelze keine geordnete kristalline Struktur aufweist. Ein Überführen der Schmelze in einen glasigen Bestandteil kann insbesondere dadurch erreicht werden, dass die Schmelze ausreichend schnell abgekühlt wird, wodurch eine Kristallisation des Schmelzmaterials verhindert wird.

Der glasige Bestandteil eignet sich besonders vorteilhaft für die Analyse des Probenmaterials, weil in diesem die homogene Mischung, die sich durch das Aufschmelzen des Probenmaterials zusammen mit dem Schmelzmittel ergibt, erhalten bleibt und dieser dabei formstabil ist.

Die Ausbildung eines glasigen Bestandteils der erstarrten Schmelze kann auch durch eine aktive Kühlung, beispielsweise mittels einer Gasströmung (z.B. Luft oder ein Schutzgas) unterstützt werden. Die erfindungsgemäße Anlage kann hierfür eine Kühlvorrichtung aufweisen. Dabei kann auch eine Regelung der aktiven Kühlung beziehungsweise der Kühlvorrichtung dahingehend vorgesehen sein, dass eine Kristallisation möglichst verhindert wird.

Als bei der Durchführung des erfindungsgemäßen Verfahrens vorteilhaft verwendbares Schmelzmittel kann beispielsweise Lithiumtetra- oder -metaborat, Natriumtetra- oder -metaborat, Natriumcarbonat, Kaliumdisulfat und/oder eine Säure, beispielsweise Borsäure, oder ein Gemisch daraus - auch unter Zugabe von Zusatzstoffen (z.B. Fließmittel wie LiBr) - eingesetzt werden.

Weiterhin bevorzugt kann vorgesehen sein, dass das Schmelzmittel mit dem Probenmaterial in einem Verhältnis von zwischen 40:1 bis 2:1, vorzugsweise von zwischen 10:1 und 2:1, vermischt wird.

In einer vorteilhaften Ausführungsform des erfindungsgemäßen Verfahrens kann vorgesehen sein, dass das Probenmaterial oder die Probenmaterial-Schmelzmittel-Mischung schichtweise (d.h. in mehreren Schichten nacheinander) aufgeschmolzen und zur Erstarrung gebracht wird. Dies kann ein relativ schnelles Erzeugen der Schmelze infolge einer exakt dosierten und sehr hohen lokalen Energieeinbringung in das Probenmaterial oder die Probenmaterial-Schmelzmittel-Mischung (beziehungsweise den für das Aufschmelzen vorgesehenen Anteil davon) ermöglichen.

Dabei kann besonders bevorzugt vorgesehen sein, dass das schichtweise Aufschmelzen und zur Erstarrung bringen der Schmelzmittel-Probenmaterial-Mischung als generatives Fertigungsverfahren zur Herstellung der Tablette (oder des entsprechenden Bestandteils davon) genutzt wird. Die erfindungsgemäße Anlage kann hierfür eine Vorrichtung zur generativen Fertigung einer Tablette aus dem gemahlenen Probenmaterial umfassen. Ein generatives Fertigungsverfahren ist dadurch gekennzeichnet, dass ein räumlicher Körper aus einem insbesondere pulverförmige Material schichtweise erzeugt wird, indem ausgehend von einer Basisschicht schichtweise Material auf diese Basisschicht aufgebracht, aufgeschmolzen und zur Erstarrung gebracht wird. Ein relevanter Vorteil eines solchen generativen Fertigungsverfahrens liegt darin, dass bedingt dadurch, dass in einer nur relativ dünnen Schicht und lokal begrenzt Material aufgeschmolzen wird, darauf verzichtet werden kann, die Schmelze während des Erstarrens in einer der vorgesehenen Form der wieder erstarrten Schmelze (d.h. in Form der Tablette oder des entsprechenden Bestandteils davon) entsprechenden Negativform aufzunehmen.

Geeignete konkrete Ausführungsformen zur Herstellung der Tablette oder eines Bestandteils davon durch ein generatives Fertigungsverfahren sind das allgemein bekannte selektive Lasersintern, das selektive Laserschmelzen und das (selektive) Elektronenstrahlschmelzen.

Ein schichtweises Aufschmelzen und zur Erstarrung bringen kann auch dadurch erfolgen, dass zunächst eine Oberflächenschicht aufgeschmolzen und zur Erstarrung gebracht wird, und anschließend eine darunter liegende (d.h. bezüglich einer das Aufschmelzen bewirkenden Strahlungsquelle durch die Oberflächenschicht räumlich getrennte) Schicht aufgeschmolzen und zur Erstarrung gebracht wird. Dies kann schrittweise fortgesetzt werden, bis die vorgesehene Stärke der herzustellenden Tablette beziehungsweise des herzustellenden Bestandteils der Tablette erreicht worden ist. Dabei kann gegebenenfalls eine unterschiedliche Absorption für die Laserstrahlung, Plasmastrahlung und/oder Elektronenstrahlung durch die Probenmaterial-Schmelzmittel-Mischung einerseits und die wieder erstarrte Schmelze andererseits ausgenutzt werden.

In einer Weiterbildung eines erfindungsgemäßen Verfahrens, bei dem das Probenmaterial oder die Probenmaterial-Schmelzmittel-Mischung aufgeschmolzen wird, kann noch vorgesehen sein, dass die erstarrte Schmelze gemahlen und anschließend in die Tablettenform überführt und dazu insbesondere verpresst wird. Es kann somit vorgesehen sein, aus der erstarrten und dann gemahlenen Schmelze eine Presstablette auszubilden. Durch eine solche Ausführungsform des erfindungsgemäßen Verfahrens wird die einfache Herstellbarkeit einer ein Probenmaterial umfassenden Tablette in Form einer Presstablette mit der für die spätere Analyse vorteilhaften Homogenisierung des Probenmaterials durch Aufschmelzen kombiniert. Bei dieser Vorgehensweise wird ausgenutzt, dass die in vorteilhafter Weise durch das Aufschmelzen erreichte Homogenisierung des Probenmaterials durch das Mahlen nicht (in einem relevanten Maße) negativ beeinflusst wird.

In einer weiteren bevorzugten Ausführungsform des erfindungsgemäßen Verfahrens kann vorgesehen sein, dass das gemahlene Probenmaterial oder die Probenmaterial-Schmelzmittel-Mischung (gegebenenfalls nach einer Zumischung eines Bindemittels) vor einem Aufschmelzen verpresst wird. Dies kann insbesondere vorteilhaft sein, wenn nicht vorgesehen ist, die gesamte Tablette in Form einer wieder erstarrten Schmelze auszubilden und somit nicht die gesamte Menge des Probenmaterials oder der Probenmaterial-Schmelzmittel-Mischung aufgeschmolzen werden soll. Somit kann insbesondere vorgesehen sein, die Tablette grundsätzlich in Form einer Presstablette auszubilden, während lediglich eine primär für die spätere Analyse vorgesehene Oberflächenschicht aufgeschmolzen und wieder zur Erstarrung gebracht wird, um die Analyse durch die vorzugsweise zu erhaltende glasige und damit amorphe Struktur sowie durch eine Homogenisierung des Probenmaterials in dieser Oberflächenschicht entsprechend zu verbessern. Dabei kann die Oberflächenschicht vorteilhafterweise eine Stärke von zwischen 30 µm und 300 µm aufweisen.

Durch ein vorzugsweise vorgesehenes Aufschmelzen des Probenmaterials oder der Probenmaterial-Schmelzmittel-Mischung mittels Laserstrahlung, Plasmastrahlung, Hochtemperatur-Infrarotstrahlung, Mikrowellenstrahlung und/oder Elektronenstrahlung kann in vorteilhafter Weise realisiert werden, dass das Aufschmelzen und/oder das Herbeiführen der Erstarrung der Schmelze (und eine dabei gegebenenfalls zum Einsatz kommende aktive Kühlung) in Echtzeit überwacht und gegebenenfalls geregelt werden kann. Die erfindungsgemäße Anlage kann hierfür eine entsprechende Vorrichtung zur Echtzeit-Überwachung eines Aufschmelzens des Probenmaterials und/oder eines Kühlens des Probenmaterials und/oder der Schmelze umfassen. Dabei kann auch ausgenutzt werden, dass eine Leistungsregelung der die Laserstrahlung, Plasmastrahlung, Hochtemperatur-Infrarotstrahlung, Mikrowellenstrahlung und/oder Elektronenstrahlung erzeugenden Vorrichtung sehr schnell beziehungsweise im Wesentlichen unmittelbar zu einer entsprechenden Regelung der in die Probenmaterial-Schmelzmittel-Mischung eingebrachten Wärmeenergie führt. Es kann somit eine sehr schnelle und exakte Regelung des Aufschmelzprozesses realisiert werden. Eine solche Echtzeit-Überwachung kann insbesondere eine Echtzeit-Temperaturregelung, beispielsweise basierend auf einer Temperaturmessung mittels eines oder mehrerer Pyrometer, umfassen. Dadurch kann beispielsweise beim Aufschmelzen die Schmelzetemperatur konstant gehalten werden, wobei beispielsweise eine Schmelzetemperatur zwischen 1000°C und 1200°C eingestellt und insbesondere eingeregelt werden kann.

Eine solche Überwachung kann vorteilhafterweise unter Verwendung von bildgebender Sensorik, wie beispielsweise einer CMOS-, Wärmebild- und/oder Infrarotkamera, und/oder eines Pyrometers erfolgen.

In einer weiterhin bevorzugten Ausführungsform des erfindungsgemäßen Verfahrens kann vorgesehen sein, dass ein Dekontaminierungsmittel in einer Mühle gemahlen und anschließend das Probenmaterial, die Probenmaterial-Schmelzmittel-Mischung oder die erstarrte Schmelze in derselben Mühle gemahlen wird. Dies kann dazu dienen, die Mühle, die gegebenenfalls zuvor schon für ein Mahlen eines andersartigen Probenmaterials genutzt wurde, zu dekontaminieren beziehungsweise gezielt mit dem anschließend zu mahlenden Probenmaterial zu kontaminieren. Dadurch kann eine Kontaminierung des Probenmaterials, die zu einer Verfälschung des Ergebnisses einer sich anschließenden Analyse dieses Probenmaterials führen könnte, möglichst ausgeschlossen werden.

Das Dekontaminierungsmittel kann vorzugsweise eine erste Teilmenge des Probenmaterials oder der Probenmaterial-Schmelzmittel-Mischung sein oder zumindest umfassen. Ebenso kann auch eine Teilmenge der erstarrten Schmelze als Dekontaminierungsmittel genutzt werden. Dies kann insbesondere dann sinnvoll sein, wenn eine andere Teilmenge der erstarrten Schmelze anschließend in der Mühle gemahlen wird, um daraus eine Presstablette auszubilden. Somit kann auch vorgesehen sein, eine Mühle mittels eines ersten Dekontaminierungsmittels, das vorzugsweise eine erste Teilmenge des Probenmaterials umfasst, für ein anschließendes Mahlen einer weiteren Teilmenge des Probenmaterials zu dekontaminieren, wobei diese weitere Teilmenge des Probenmaterials anschließend mit dem Schmelzmittel vermischt und erfindungsgemäß aufgeschmolzen wird. Anschließend kann in derselben oder einer anderen Mühle eine erste Teilmenge der erstarrten Schmelze zur Dekontaminierung der Mühle gemahlen und anschließend eine weitere Teilmenge der erstarrten Schmelze gemahlen werden, um daraus eine Presstablette auszubilden.

Alternativ oder zusätzlich kann das Dekontaminierungsmittel auch ein probenmaterialfremdes Material, wie insbesondere Quarzsand, Korund und/oder ein beliebiges feuerfestes Material (auch schamottehaltiges Material) sein oder umfassen.

In einer Ausführungsform des erfindungsgemäßen Verfahrens kann vorgesehen sein, dass das gemahlene Dekontaminierungsmittel verworfen wird, so dass es zumindest nicht weiter im Rahmen des erfindungsgemäßen Verfahrens genutzt wird. Dabei kann insbesondere vorgesehen sein, dass das Dekontaminierungsmittel entsorgt wird.

Möglich ist auch, dass das/die gemahlene(n) Dekontaminierungsmittel verpresst und als Tragschicht für zu verpressendes Probenmaterial oder zu verpressende Probenmaterial-Schmelzmittel-Mischung und/oder eine zu verpressende (Teil-) Menge von erstarrter und anschließend gemahlener Schmelze genutzt wird/werden. In diesem Fall kann eine Auswahl des Dekontaminierungsmittels (oder zumindest eines Bestandteils davon) auch anhand einer Eignung als Tragschicht erfolgen. Ein Verpressen des/der Dekontaminierungsmittel(s) kann vorzugsweise (jeweils) in einem Formring erfolgen. Dessen Höhe kann dabei vorteilhafterweise größer, vorzugsweise mindestens doppelt so groß wie die Schichtstärke der Tragschicht sein. Die Einheit aus Tragschicht und Formring kann dann als gefäßartige Negativform (zu der vorgesehenen Tablettenform) für die anschließend darin einzubringende Menge des Probenmaterials oder der Probenmaterial-Schmelzmittel-Mischung oder einer Menge von daraus ausgebildeter gemahlener erstarrte Schmelze dienen, die darin auch weiterbearbeitet, insbesondere verpresst und/oder aufgeschmolzen werden kann.

Vorteilhafterweise kann der Formring zumindest teilweise aus Edelstahl oder einem Refraktärmetall (oder auch Cermet) mit geringem thermischem Ausdehnungskoeffizienten bestehen.

Um einen sicheren Halt der Presstablette innerhalb des Formrings zu gewährleisten kann vorzugsweise vorgesehen sein, dass dieser mindestens eine innenseitige Vertiefung ausbildet, in die das Probenmaterial oder die Probenmaterial-Schmelzmittel-Mischung (jeweils in pulvriger oder glasiger Form) und/oder Dekontaminierungsmittel beim jeweiligen Verpressen eindringen kann, wodurch sich eine formschlüssige Verbindung zwischen der herzustellenden Presstablette und dem Formring ausbilden kann. Alternativ kann der Formring an einer Oberseite innen eine Nut aufweisen oder aber innen insgesamt konisch zulaufen.

Bei dem Probenmaterial kann es sich insbesondere um ein oder mehrere natürliche Gesteine bzw. Erze, wie beispielsweise Silikate, Karbonate, Sulfate, Sulfide, Salze und/oder Oxide handeln. Weiterhin kann das Probenmaterial insbesondere industrielle Prozessprodukte, wie beispielsweise Schlacke, Flugasche und/oder Zementklinker, umfassen.

Die unbestimmten Artikel ("ein", "eine", "einer" und "eines"), insbesondere in den Patentansprüchen und in der die Patentansprüche allgemein erläuternden Beschreibung, sind als solche und nicht als Zahlwörter zu verstehen. Entsprechend damit konkretisierte Komponenten sind somit so zu verstehen, dass diese mindestens einmal vorhanden sind und mehrfach vorhanden sein können.

Die Erfindung wird nachfolgend anhand von in den Zeichnungen dargestellten Ausführungsbeispielen näher erläutert. In den Zeichnungen zeigt
- Fig. 1:: schematisch eine im Rahmen einer ersten Ausführungsform eines erfindungsgemäßen Verfahrens hergestellte Tablette;
- Fig. 2:: schematisch eine im Rahmen einer zweiten Ausführungsform eines erfindungsgemäßen Verfahrens hergestellte Tablette;
- Fig. 3:: schematisch eine Variante zur Herstellung einer Presstablette im Rahmen eines erfindungsgemäßen Verfahrens und eine dabei genutzte Vorrichtung;
- Fig. 4:: schematisch eine andere Variante zur Herstellung einer Presstablette im Rahmen eines erfindungsgemäßen Verfahrens und eine dabei genutzte Vorrichtung;
- Fig. 5:: schematisch einen ersten Schritt bei der Analyse einer ein Probenmaterial umfassenden Tablette im Rahmen eines erfindungsgemäßen Verfahrens; und
- Fig. 6:: schematisch einen zweiten Schritt bei der Analyse der Tablette gemäß der Fig. 5.

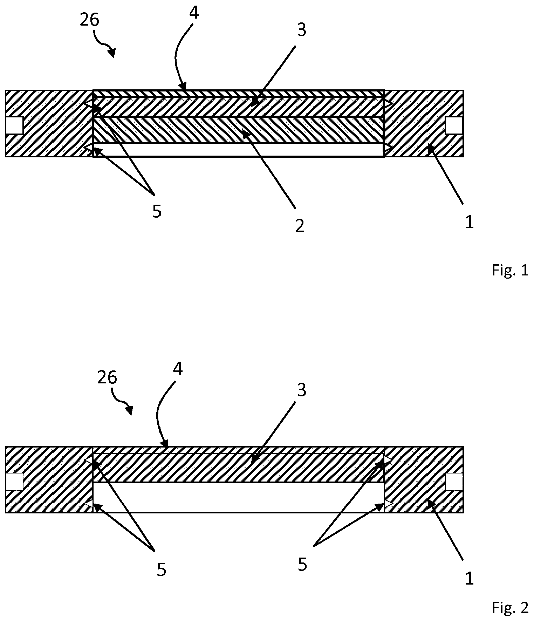

Die Fig. 1 zeigt eine ein Probenmaterial umfassende Tablette 26, die im Rahmen einer ersten Ausführungsform eines erfindungsgemäßen Verfahrens hergestellt wurde.

Bei dieser Ausführungsform eines erfindungsgemäßen Verfahrens wird zunächst eine erste Teilmenge (z.B. 5 bis 6 g) des aufzubereitenden und zu analysierenden Probenmaterials in eine Feinmühle (nicht dargestellt) dosiert und dort als Spülbeziehungsweise Vorprobe gemahlen. Hierdurch wird die Feinmühle gezielt mit dem aufzubereitenden Probenmaterial kontaminiert. Diese erste Teilmenge des Probenmaterials dient somit als Dekontaminierungsmittel.

Die als Dekontaminierungsmittel genutzte Teilmenge des Probenmaterials wird nach dem Mahlen in der Feinmühle mittels einer Tablettenpresse (nicht dargestellt) in einen Formring 1 aus beispielsweise Edelstahl gepresst (vgl. Fig. 1), um eine Tragschicht 2 für eine weitere Schicht 3 aus einer Mischung einer zweiten Teilmenge des Probenmaterials und einem Schmelzmittel (z.B. ein Schmelzsalz) auszubilden. Diese weitere Schicht 3 beziehungsweise ein Bestandteil davon dient einer nachträglichen Analyse des Probenmaterials. Die Tragschicht 2 ist dafür infolge einer möglichen Kontaminierung durch in der Feinmühle enthaltene Verunreinigungen nicht geeignet. Für die Tragschicht 2 kann beispielsweise eine Schichtstärke von drei bis vier Millimetern vorgesehen sein. Diese Schichtstärke kann ca. 50% der Höhe des Formrings 1 entsprechen. Nach einer Reinigung des Formrings 1 und der Tragschicht 2 wird die aus diesen beiden Komponenten ausgebildete Einheit um 180° (bezüglich einer Radialachse) gedreht, so dass diese Einheit ein nach oben geöffnetes Gefäß ausbildet, in das die weitere Teilmenge des Probenmaterials eingebracht werden kann.

Anschließend wird eine weitere Teilmenge des Probenmaterials in die Feinmühle dosiert und dort in einem ersten Mahlschritt gemahlen. Die Drehzahl, mit der die Feinmühle betrieben wird, und die Mahldauer können dabei in Abhängigkeit von dem zu bearbeitenden Probenmaterial eingestellt werden. Nach dem Abschluss des ersten Mahlschritts wird ein Schmelzmittel (z.B. Lithiumtetraborat) gemäß einem vorgegebenen Mischungsverhältnis in diese gemahlene Teilmenge des Probenmaterials gemischt (z.B. mit einem Mischungsverhältnis von 1:5, d.h. bei einer Menge der weiteren Teilmenge des Probenmaterials von 2 g eine Menge des Schmelzmittels von 10 g). Anschließend wird diese Probenmaterial-Schmelzmittel-Mischung in einem zweiten Mahlschritt in der Feinmühle gemahlen und dabei möglichst optimal durchgemischt. Die Probenmaterial-Schmelzmittel-Mischung wird dann in das von dem Formring 1 und der Tragschicht 2 ausgebildete Gefäß eingebracht und in der Tablettenpresse verpresst. Die so entstandene Presstablette 26 besteht zunächst aus zwei Schichten, nämlich der Tragschicht 2, die aus der gegebenenfalls kontaminierten ersten Teilmenge des Probenmaterials besteht, sowie aus der weiteren, bei der sich anschließenden Analyse zu berücksichtigenden Schicht 3, die aus der verpressten Probenmaterial-Schmelzmittel-Mischung besteht.

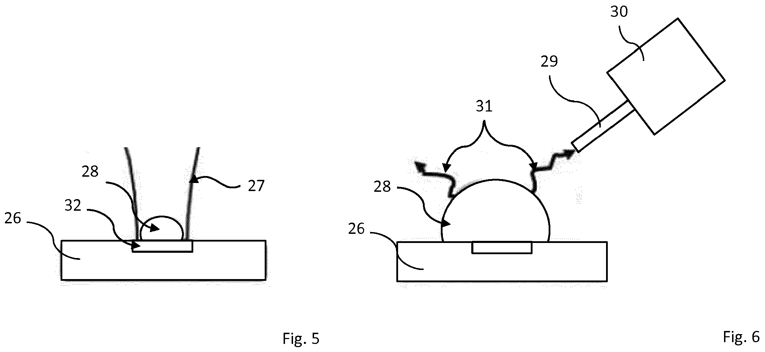

Die freie Oberfläche dieser weiteren Schicht 3 wird anschließend mittels eines Lasers (in der Fig. 1 nicht dargestellt) bestrahlt und dabei aufgeschmolzen. Die entsprechende Oberfläche wird dabei vollständig oder in kleinen Abschnitten mittels kontinuierlicher oder gepulster Laserstrahlung (z.B. mittels eines Faserlasers) umgeschmolzen beziehungsweise aufgeschlossen. Nach einem Erstarren dieser Schmelze durch Abkühlen umfasst die herzustellende Tablette 26 eine glasige Schicht 4, die durch ein besonders homogenes Materialgefüge gekennzeichnet ist. Diese glasige Schicht 4 eignet sich in vorteilhafter Weise für eine anschließende Analyse des Probenmaterials, die erfindungsgemäß im Rahmen einer Laseremissionsspektroskopie durchgeführt wird(vgl. Fig. 5 und 6).

Bei einer möglichen Variante wird zum Aufschmelzen der Probenmaterial-Schmelzmittel-Mischung in der freien Oberfläche der Schicht 3 mit einem Laserstrahl Punkt für Punkt (Fläche für Fläche) der Oberfläche der weiteren Schicht 3 abgerastert. Dabei werden sehr kleine "Schmelzpfützen" erzeugt, die kurze Zeit später wieder glasig erstarren. Durch den fließenden Prozess infolge des Verfahrens des Lasers wird die gesamte Oberfläche homogen aufgeschlossen und zu einer glasigen Schicht 4 umgewandelt.

Der Sinn des Schmelzaufschlusses ist es, das Probenmaterial zusammen mit einem Schmelzmittel in eine homogene Schmelze zu überführen, die beim Abkühlen glasig erstarrt. Durch den Aufschluss werden Einflüsse auf die Messergebnisse der Lasermissionsspektroskopie beseitigt oder zumindest verringert, die beispielsweise durch die Korngrößenverteilung und den mineralogischen Originalzustand des Probenmaterials begründet sein können. Der Aufschluss führt zu einer einheitlichen Bindungsform der Elemente und mindert durch Verdünnung die gegenseitige Beeinflussung der Elemente durch Sekundäranregung und Absorption (Matrixeffekte).

Das Umschmelzen ist ein chemischer Vorgang. Das Schmelzmittel zersetzt dabei die Verbindungen des Probenmaterials und wandelt diese chemisch um. Aus Silikaten, Aluminaten, Carbonaten und Sulfaten des Probenmaterials (soweit vorhanden) werden so beispielsweise Borate. Durch ein Umschmelzen beziehungsweise Aufschmelzen von beispielsweise Fe₂O₃, Fe₃O₄, FeO, FeCl₂, FeS₂, FeCO₃ mit Tetraborat entstehen Fe-Borate. Durch Umwandlung in Fe-Borate werden die Einflüsse der chemischen Bindung minimiert.

In einer alternativen Ausführungsform (gemäß der Fig. 2) des zuvor beschriebenen Verfahrens ist vorgesehen, dass keine Tragschicht 2 erzeugt wird. Vielmehr wird eine Presstablette 26 ausschließlich aus der Probenmaterial-Schmelzmittel-Mischung ausgebildet. Dazu wird zunächst wieder eine erste Teilmenge des Probenmaterials als Spülprobe beziehungsweise Dekontaminierungsmittel gemahlen, anschließend jedoch verworfen. Nach der erfolgten Dekontaminierung der Mühle wird wiederum eine zweite Teilmenge des Probenmaterials (z.B. 2 g) in zwei Mahlschritten aufbereitet. Im ersten Mahlvorgang wird lediglich diese zweite Teilmenge des Probenmaterials gemahlen. Für den zweiten Mahlschritt wird dann in einem möglichst exakten Verhältnis von beispielsweise 1:5 (Probenmaterial zu Schmelzmittel) ein Schmelzmittel hinzugegeben und gemeinsam mit dem Probenmaterial gemahlen, um eine möglichst optimale Durchmischung dieser Komponenten zu erreichen. Die Mischung aus dem Probenmaterial und dem Schmelzmittel wird dann in eine Tablettenpresse (nicht dargestellt) überführt. Dort wird die Mischung zu einer der Form der herzustellenden Tablette 26 im Wesentlichen entsprechenden Schicht 3 gepresst. Anschließend kann das Aufschmelzen und das Erstarren lassen einer Oberflächenschicht der Presstablette 26 entsprechend der zuvor beschriebenen Ausführungsform eines erfindungsgemäßen Verfahrens durchgeführt werden, um eine glasige Schicht 4 auszubilden.

Der im Rahmen der Durchführung der zuvor beschriebenen Verfahren genutzte Formring 1 weist an seiner Innenseite zwei umlaufende V-förmige Vertiefungen 5 auf. Dabei befindet sich eine der Vertiefungen 5 in der oberen axialen Hälfte und die andere Vertiefung 5 in der unteren axialen Hälfte des Formrings 1. Der Formring 1 weist beispielsweise eine (axiale) Höhe von 8,5 mm auf. Eine der Vertiefungen 5 befindet sich ausgehend von einem der axialen Enden des Formrings 1 beispielsweise auf einer axialen Höhe von 2,5 mm und die andere Vertiefung (ausgehend von demselben axialen Ende des Formrings 1) auf einer axialen Höhe von 6 mm.

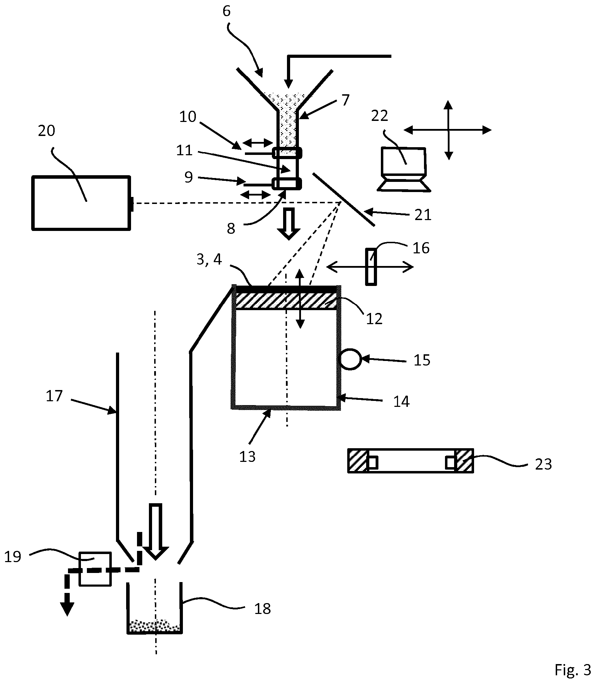

Die Fig. 3 zeigt schematisch die Herstellung einer ein Probenmaterial umfassenden Tablette 26 im Rahmen eines erfindungsgemäßen Verfahrens in einer dritten Ausführungsform sowie eine dabei genutzte Vorrichtung.

Dieses Verfahrens lehnt sich an die Methode des selektiven Laserschmelzens (SLM) an. Das dabei verarbeitete Pulver besteht aus einer Mischung aus einem Probenmaterial und einem Schmelzmittel. Dieses Pulver wird entsprechend dem Vorgehen bei den beiden zuvor beschriebenen Ausführungsformen erfindungsgemäßer Verfahren aufbereitet, d.h. nach einer möglichen Dekontaminierung einer Feinmühle (nicht dargestellt) wird eine Teilmenge des Probenmaterials in einem ersten Mahlschritt in der Feinmühle gemahlen, darauf folgend mit dem Schmelzmittel vermischt und anschließend die Probenmaterial-Schmelzmittel-Mischung in einem zweiten Mahlschritt in der Feinmühle durchmischt.

Anschließend wird eine ausreichende Menge der Probenmaterial-Schmelzmittel-Mischung über einen Einlass 6 einem Dosierer 7 der für die Durchführung dieser Ausführungsform eines erfindungsgemäßen Verfahrens genutzten Vorrichtung zugeführt und hier bevorratet. Im Bereich eines Auslasses 8 umfasst der Dosierer 7 zwei parallel zueinander beabstandete Dosierschieber 9, 10, die in bekannter Weise eine Separierung eines definierten Volumens der Probenmaterial-Schmelzmittel-Mischung von der in dem Dosierer 7 vorgehaltenen Gesamtmenge der Probenmaterial-Schmelzmittel-Mischung ermöglichen, indem bei geschlossenem unterem Dosierschieber 9 der obere Dosierschieber 10 geöffnet wird, so dass Probenmaterial-Schmelzmittel-Mischung in den zwischen den Dosierschiebern 9, 10 ausgebildeten Dosierraum 11 fallen kann. Durch ein Schließen des oberen Dosierschiebers 10 wird dann eine dem Volumen des Dosierraums 11 entsprechende Teilmenge der Probenmaterial-Schmelzmittel-Mischung von der in dem Dosierer 7 vorgehaltenen Restmenge der Probenmaterial-Schmelzmittel-Mischung separiert. Diese separierte Teilmenge kann dann durch ein Öffnen des unteren Dosierschiebers 9 aus dem Dosierer 7 ausgebracht werden.

Eine derart ausgebrachte Teilmenge der Probenmaterial-Schmelzmittel-Mischung fällt auf einen Teller 12 einer unter dem Dosierer 7 angeordneten Aufnahmevorrichtung 13. Der Teller 12 ist innerhalb einer Führung 14 dieser Aufnahmevorrichtung 13 in vertikaler Richtung verfahrbar. Die Aufnahmevorrichtung 13 umfasst weiterhin einen Vibrator 15, mittels dessen die Aufnahmevorrichtung 13 in Schwingung versetzt werden kann. Dies dient einer Auflockerung der auf dem Teller 12 aufliegenden Teilmenge der Probenmaterial-Schmelzmittel-Mischung sowie einer ersten flächigen Verteilung dieser Teilmenge auf dem Teller 12.

Anschließend wird mittels eines Verteilschiebers 16 die Probenmaterial-Schmelzmittel-Mischung in einer möglichst gleichmäßig starken Schicht auf dem Teller 12 verteilt und dabei überschüssige Probenmaterial-Schmelzmittel-Mischung über einen Probenverwurf 17 in einen Probenbecher 18 geleitet. Diese überschüssige Probenmaterial-Schmelzmittel-Mischung kann zur Wiederverwendung aus dem Probenbecher 18 zurück in den Dosierer 7 gefördert werden. Dazu kann der Probenbecher 18 mittels einer automatischen Handhabungsvorrichtung, wie beispielsweise einem Roboter (nicht dargestellt), entnommen und zu dem Einlass 6 des Dosierers 7 bewegt werden. Alternativ kann auch vorgesehen sein, dass die überschüssige Probenmaterial-Schmelzmittel-Mischung aus dem Probenbecher 18 entfernt, beispielsweise mittels einer Absaugvorrichtung 19 abgesaugt, und anschließend gegebenenfalls entsorgt wird.

Die geglättet auf dem Teller 12 der Aufnahmevorrichtung 13 aufliegende Schicht der Probenmaterial-Schmelzmittel-Mischung wird anschließend mittels eines Lasers 20 bestrahlt und dabei selektiv aufgeschmolzen. Hierzu ist ein automatisiert schwenkbarer Spiegel 21 vorgesehen, durch dessen Schwenkbewegung ermöglicht wird, einen definierten Bereich der Schicht der Probenmaterial-Schmelzmittel-Mischung mittels eines durch den Laser 20 erzeugten Laserstrahls nach und nach zu bestrahlen und dadurch aufzuschmelzen (alternativ auch ohne Spiegel möglich, direkter Energieeintrag). Nach einem Erstarren der so erzeugten Schmelze entsteht eine glasige Schicht. Bei dieser glasigen Schicht kann es sich bereits um die herzustellende Tablette 26 handeln. Möglich ist aber auch, schichtweise eine Mehrzahl solcher (stoffschlüssig miteinander verbundenen) glasigen Schichten übereinander auszubilden, indem aufeinanderfolgend jeweils ein Aufbringen einer dosierten Teilmenge der Probenmaterial-Schmelzmittel-Mischung auf den Teller 12 der Aufnahmevorrichtung 13, ein Verteilen beziehungsweise Glätten dieser Teilmenge und ein Aufschmelzen und Erstarren Lassen dieser Teilmenge durchgeführt werden, wobei für jeden Zyklus der Teller 12 um eine Distanz, die der vorgesehenen Schichtstärke der auszubildenden glasigen Schicht im Wesentlichen entspricht, nach unten verfahren wird.

Die so erzeugte(n) glasige(n) Schicht(en) kann/können dann noch optional auf eine Tragschicht (nicht dargestellt) aufgebracht werden, um die Formstabilität der herzustellenden Tablette 26 zu erhöhen.

Mittels einer Transportvorrichtung 22, beispielsweise einem Sauggreifelement, kann/können die Tablette 26 beziehungsweise die glasige(n) Schicht(en) dann aus der Aufnahmevorrichtung 13 entnommen und in einen (nicht dargestellten) Analysator , beispielsweise einen Röntgenfluoreszenzanalysator, überführt werden. Gegebenenfalls kann auch vorgesehen sein, die so erzeugte(n) glasige(n) Schicht(en) (gegebenenfalls in Verbindung mit der Tragschicht) zuvor in einen Probenträger 23 einzusetzen, um die Handhabbarkeit der erzeugten Tablette 26 im Rahmen der Analyse zu verbessern.

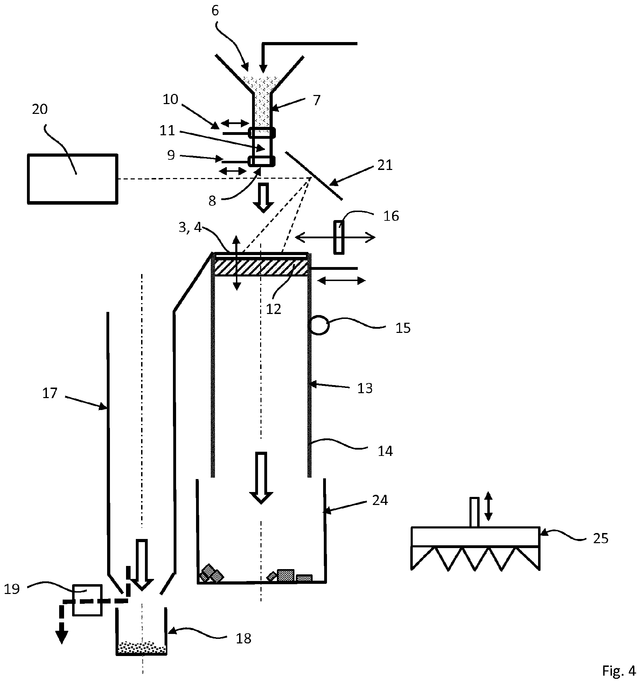

Die Fig. 4 zeigt schematisch die Herstellung einer ein Probenmaterial umfassenden Tablette 26 im Rahmen eines erfindungsgemäßen Verfahrens in einer vierten Ausführungsform sowie eine dabei genutzte Vorrichtung.

Auch diese Ausfuhrungsform eines erfindungsgemäßen Verfahrens lehnt sich an die Methode des selektiven Laserschmelzens (SLM) an. Das dabei verarbeitete Pulver besteht wiederum aus einer Mischung des Probenmaterials mit einem Schmelzmittel. Dieses Pulver wird entsprechend dem Vorgehen bei den zuvor beschriebenen Ausführungsformen erfindungsgemäßer Verfahren aufbereitet, d.h. nach einer möglichen Dekontaminierung einer Feinmühle (nicht dargestellt) wird eine Teilmenge des Probenmaterials in einem ersten Mahlschritt in der Feinmühle gemahlen, darauf folgend mit dem Schmelzmittel vermischt und anschließend die Probenmaterial-Schmelzmittel-Mischung in einem zweiten Mahlschritt in der Feinmühle durchmischt.

Anschließend wird eine ausreichende Menge der Probenmaterial-Schmelzmittel-Mischung über einen Einlass 6 einem Dosierer 7 der für die Durchführung dieser Ausführungsform eines erfindungsgemäßen Verfahrens genutzten Vorrichtung zugeführt und hier bevorratet. Im Bereich eines Auslasses 8 umfasst der Dosierer 7 zwei parallel zueinander beabstandete Dosierschieber 9, 10, die in bekannter Weise eine Separierung eines definierten Volumens der Probenmaterial-Schmelzmittel-Mischung von der in dem Dosierer 7 vorgehaltenen Gesamtmenge der Probenmaterial-Schmelzmittel-Mischung ermöglichen, indem bei geschlossenem unterem Dosierschieber 9 der obere Dosierschieber 10 geöffnet wird, so dass Probenmaterial-Schmelzmittel-Mischung in den zwischen den Dosierschiebern 9, 10 ausgebildeten Dosierraum 11 fallen kann. Durch ein Schließen des oberen Dosierschiebers 10 wird dann eine dem Volumen des Dosierraums 11 entsprechende Teilmenge der Probenmaterial-Schmelzmittel-Mischung von der in dem Dosierer 7 vorgehaltenen Restmenge der Probenmaterial-Schmelzmittel-Mischung separiert. Diese separierte Teilmenge kann dann durch ein Öffnen des unteren Dosierschiebers 9 aus dem Dosierer 7 ausgebracht werden.

Eine derart ausgebrachte Teilmenge der Probenmaterial-Schmelzmittel-Mischung fällt auf einen Teller 12 einer unter dem Dosierer 7 angeordneten Aufnahmevorrichtung 13. Der Teller 12 ist innerhalb einer rohrförmigen Führung 14 dieser Aufnahmevorrichtung 13 in vertikaler und horizontaler Richtung verfahrbar. Die Aufnahmevorrichtung 13 umfasst weiterhin einen Vibrator 15, mittels dessen die Aufnahmevorrichtung 13 in Schwingung versetzt werden kann. Dies dient einer Auflockerung der auf dem Teller 12 aufliegenden Teilmenge der Probenmaterial-Schmelzmittel-Mischung sowie einer ersten flächigen Verteilung dieser Teilmenge auf dem Teller 12.

Anschließend wird mittels eines Verteilschiebers 16 die Probenmaterial-Schmelzmittel-Mischung in einer möglichst gleichmäßig starken Schicht auf dem Teller 12 verteilt und dabei überschüssige Probenmaterial-Schmelzmittel-Mischung über einen Probenverwurf 17 in einen Probenbecher 18 geleitet. Diese überschüssige Probenmaterial-Schmelzmittel-Mischung kann zur Wiederverwendung aus dem Probenbecher 18 zurück in den Dosierer 7 gefördert werden. Dazu kann der Probenbecher 18 mittels einer automatischen Handhabungsvorrichtung, wie beispielsweise einem Roboter (nicht dargestellt), entnommen und zu dem Einlass 6 des Dosierers 7 bewegt werden. Alternativ kann auch vorgesehen sein, dass die überschüssige Probenmaterial-Schmelzmittel-Mischung aus dem Probenbecher 18 entfernt, beispielsweise mittels einer Absaugvorrichtung 19 abgesaugt, und anschließend gegebenenfalls entsorgt wird.

Die geglättet auf dem Teller 12 der Aufnahmevorrichtung 13 aufliegende Schicht der Probenmaterial-Schmelzmittel-Mischung wird anschließend mittels eines Lasers 20 bestrahlt und dabei selektiv aufgeschmolzen. Hierzu ist ein automatisiert schwenkbarer Spiegel 21 vorgesehen, durch dessen Schwenkbewegung ermöglicht wird, einen definierten Bereich der Schicht der Probenmaterial-Schmelzmittel-Mischung mittels eines durch den Laser 20 erzeugten Laserstrahls nach und nach zu bestrahlen und dadurch aufzuschmelzen (alternativ auch ohne Spiegel möglich, direkter Energieeintrag). Nach einem Erstarren entsteht eine aufgeschmolzene und abgekühlte Schicht. Gegebenenfalls kann vorgesehen sein, schichtweise eine Mehrzahl solcher (stoffschlüssig miteinander verbundenen) Schichten übereinander auszubilden, indem aufeinanderfolgend jeweils ein Aufbringen einer dosierten Teilmenge der Probenmaterial-Schmelzmittel-Mischung auf den Teller 12 der Aufnahmevorrichtung 13, ein Verteilen beziehungsweise Glätten dieser Teilmenge und ein Aufschmelzen und erstarren Lassen dieser Teilmenge durchgeführt werden, wobei für jeden Zyklus der Teller 12 um eine Distanz, die der vorgesehenen Schichtstärke der gerade auszubildenden aufgeschmolzenen Schicht im Wesentlichen entspricht, vertikal (nach unten) verfahren wird.

Nach der Ausbildung einer oder mehrerer aufgeschmolzener, abgekühlter Schichten wird der Teller 12 horizontal verfahren, wodurch der oder die aufgeschmolzenen, abgekühlten Schichten in einen Aufnahmebehälter 24 fallen. Sobald in dem Aufnahmebehälter 24 eine ausreichende Menge an aufgeschmolzenem, abgekühltem Material (d.h. die aufgeschmolzene(n) Schicht(en) 4, gegebenenfalls in Fragmente zerbrochen) vorliegt, wird der Aufnahmebehälter 24 mittels einer automatischen Handhabungsvorrichtung, beispielsweise einem Roboter entnommen und einer Vorzerkleinerungsvorrichtung 25 zugeführt. Das aufgeschmolzene, abgekühlte Material kann dazu beispielsweise in dem Aufnahmebehälter 24 mittels eines Mörsers oder einer geeigneten Stoßvorrichtung vorzerkleinert werden. Anschließend werden die vorzerkleinerten Partikel einer Feinmühle zugeführt und dort gemahlen. Nach dem Mahlen wird aus dem zuvor aufgeschmolzenen, abgekühlten Material in einer Tablettenpresse eine analysenfertige Presstablette 26 erstellt. Hierzu kann das aufgeschmolzene, abgekühlte und dann vermahlene Material in einem Formring verpresst werden, wie dies anhand der Fig. 1 und 2 beschrieben worden ist.

In allen beschriebenen Ausführungsformen kann anstelle eines Aufschmelzens der Probenmaterial-Schmelzmittel-Mischung mittels Laserstrahlung auch ein Aufschmelzen mittels Plasmastrahlung, Hochtemperatur-Infrarotstrahlung, Mikrowellenstrahlung oder Elektronenstrahlung durchgeführt werden, ohne dass sich die übrigen beschriebenen Verfahrensschritte dafür ändern müssten. Unter Umständen können einzelne Verfahrensschritte auch unter Schutzgas ablaufen (z.B. Argon).

Grundsätzlich in allen erfindungsgemäßen Verfahren und so auch bei den oben beschriebenen Ausführungsformen erfindungsgemäßer Verfahren können vorteilhafterweise zusätzliche Verfahrensschritte durchgeführt werden, die insbesondere der Sicherstellung einer hinreichenden Qualität der herzustellenden Tablette 26 dienen. Dabei kann insbesondere vorgesehen sein, dass das Aufschmelzen der Probenmaterial-Schmelzmittel-Mischung und das wieder Erstarren lassen der Schmelze regelmäßig oder kontinuierlich überwacht werden, insbesondere mittels einer Messung der Temperatur der Schmelze, beispielsweise mittels eines Pyrometers (nicht dargestellt) und/oder mittels einer Überwachung der Wärmeverteilung der Schmelze, beispielsweise mittels einer Wärmebildkamera (nicht dargestellt) und entsprechender Bildverarbeitungssoftware. Ebenso kann eine Beobachtung des Schmelzvorganges mittels einer Kamera (insbesondere CMOS; nicht dargestellt) erfolgen. Ebenfalls mittels einer Kamera (nicht dargestellt) kann auch eine Überwachung der Ausbildung der aufgeschmolzenen Schicht beziehungsweise eine Qualitätsprüfung des dabei ausgebildeten Glases durchgeführt werden. Einige oder sämtliche dieser Schritte können vorteilhafterweise auch in einer Ausgestaltung als Echtzeitüberwachung (Online-System) vorgesehen sein.

Die Fig. 5 und 6 zeigen zwei Schritte bei der Analyse einer ein Probenmaterial umfassenden Tablette 26 im Rahmen eines erfindungsgemäßen Verfahrens.

Für die Analyse, die erfindungsgemäß mittels Laseremissionsspektroskopie durchgeführt wird, wird ein sehr kurzer Puls energiereicher Laserstrahlung 27 punktuell auf die zu untersuchenden Oberfläche der Tablette 26 fokussiert. Die hohe Leistungsdichte in dem fokussierten Abschnitt der Oberfläche der Tablette 26 führt zu einer Erhitzung einer Teilmenge 32 des dortigen Materials auf in der Regel Werte von einigen 10.000°C. Diese hohen Temperaturen bewirken die Ausbildung eines Licht emittierenden Plasmas 28, wobei diese Lichtemission 31 charakteristisch für das zu untersuchende Material der Tablette 26 ist. Für die Analyse wird die Lichtemission 31 des Plasmas über beispielsweise eine optische Faser 29 einem Spektrometer zugeführt, so dass aus dem Spektrum der Lichtemission 31 des erzeugten Plasmas 28 die atomare Zusammensetzung des Materials qualitativ und quantitativ bestimmt werden kann.

### Bezugszeichen:

- 1: Formring
- 2: Tragschicht
- 3: Schicht aus einer Probenmaterial-Schmelzmittel-Mischung
- 4: Glasige bzw. aufgeschmolzene, abgekühlte Schicht
- 5: Vertiefung
- 6: Einlass des Dosierers
- 7: Dosierer
- 8: Auslass des Dosierers
- 9: unterer Dosierschieber
- 10: oberer Dosierschieber
- 11: Dosierraum
- 12: Teller der Aufnahmevorrichtung
- 13: Aufnahmevorrichtung
- 14: Führung der Aufnahmevorrichtung
- 15: Vibrator
- 16: Verteilschieber
- 17: Probenverwurf
- 18: Probenbecher
- 19: Absaugvorrichtung
- 20: Laser
- 21: Spiegel
- 22: Transportvorrichtung
- 23: Probenträger
- 24: Aufnahmebehälter
- 25: Vorzerkleinerungvorrichtung
- 26: (Press-)Tablette
- 27: Laserstrahlung
- 28: Plasma
- 29: optische Faser
- 30: Spektrometer
- 31: Lichtemission
- 32: Teilmenge des Materials der Tablette

## Patentansprüche

1. Verfahren zur Analyse eines Probenmaterials, wobei das Probenmaterial gemahlen, zu einer Tablette geformt und anschließend analysiert wird, wobei die Analyse mittels Laseremissionsspektroskopie durchgeführt wird und wobei das Probenmaterial zumindest teilweise mit einem Schmelzmittel vermischt wird, **dadurch gekennzeichnet, dass** das Probenmaterial in einem ersten Mahlschritt vorgemahlen und nach dem Vermischen mit dem Schmelzmittel in einem weiteren Mahlschritt weitergemahlen wird.

2. Verfahren gemäß Anspruch 1, **dadurch gekennzeichnet, dass** eine Oberfläche der Tablette im Rahmen der Laseremissionsspektroskopie an mehreren Stellen mittels eines Lasers beaufschlagt wird.

3. Verfahren gemäß einem der vorhergehenden Ansprüche, **dadurch gekennzeichnet, dass** das Probenmaterial oder die Probenmaterial-Schmelzmittel-Mischung zumindest teilweise vor der Analyse aufgeschmolzen wird.

4. Verfahren gemäß Anspruch 3, **dadurch gekennzeichnet, dass** eine Erstarrung der Schmelze herbeigeführt wird, wobei die erstarrte Schmelze die Tablettenform bereits aufweist oder in die Tablettenform überführt wird.

5. Verfahren gemäß Anspruch 3 oder 4, **dadurch gekennzeichnet, dass** das Probenmaterial oder die Probenmaterial-Schmelzmittel-Mischung während des Aufschmelzens gekühlt wird.

6. Verfahren gemäß einem der Ansprüche 3 bis 5, **dadurch gekennzeichnet, dass** zumindest ein für das Aufschmelzen vorgesehener Anteil des Probenmaterials oder der Probenmaterial-Schmelzmittel-Mischung vor dem Aufschmelzen vorgewärmt und/oder während des Aufschmelzens ergänzend erwärmt wird.

7. Verfahren gemäß einem der Ansprüche 3 bis 6, **dadurch gekennzeichnet, dass** das Probenmaterial oder die Probenmaterial-Schmelzmittel-Mischung mittels Laserstrahlung, Plasmastrahlung, Hochtemperatur-Infrarotstrahlung, Mikrowellenstrahlung und/oder Elektronenstrahlung aufgeschmolzen wird.

8. Verfahren gemäß Anspruch 6 und 7, **dadurch gekennzeichnet, dass** zumindest der für das Aufschmelzen vorgesehene Anteil des Probenmaterials oder der Probenmaterial-Schmelzmittel-Mischung mittels Laserstrahlung vorgewärmt wird und das Probenmaterial oder die Probenmaterial-Schmelzmittel-Mischung mittels Laserstrahlung desselben Lasers aufgeschmolzen wird.

9. Verfahren gemäß Anspruch 7 oder 8, **dadurch gekennzeichnet, dass** das Probenmaterial oder die Probenmaterial-Schmelzmittel-Mischung zumindest teilweise mittels Laserstrahlung aufgeschmolzen wird, wobei dazu derselbe Laser wie für die Durchführung der sich anschließenden Laseremissionsspektroskopie verwendet wird.

10. Verfahren gemäß einem der Ansprüche 3 bis 9, **dadurch gekennzeichnet, dass** das Probenmaterial oder die Probenmaterial-Schmelzmittel-Mischung selektiv aufgeschmolzen wird.

11. Verfahren gemäß Anspruch 4 oder einem der von Anspruch 4 abhängigen Ansprüche, **dadurch gekennzeichnet, dass** die Erstarrung derart herbeigeführt wird, dass ein glasiger Bestandteil der Tablette ausgebildet wird.

12. Verfahren gemäß Anspruch 4 oder einem der von Anspruch 4 abhängigen Ansprüche, **dadurch gekennzeichnet, dass** das Probenmaterial oder die Probenmaterial-Schmelzmittel-Mischung schichtweise aufgeschmolzen und zur Erstarrung gebracht wird.

13. Verfahren gemäß Anspruch 4 oder einem der von Anspruch 4 abhängigen Ansprüche, **dadurch gekennzeichnet, dass** die erstarrte Schmelze gemahlen und anschließend in die Tablettenform überführt wird.

14. Verfahren gemäß Anspruch 4 oder einem der von Anspruch 4 abhängigen Ansprüche, **dadurch gekennzeichnet, dass** das Aufschmelzen und/oder das Herbeiführen der Erstarrung der Schmelze in Echtzeit überwacht wird.

15. Verfahren gemäß Anspruch 14, **dadurch gekennzeichnet, dass** die Überwachung unter Verwendung von bildgebender Sensorik oder eines Pyrometers durchgeführt wird.

16. Verfahren gemäß einem der vorhergehenden Ansprüche, **dadurch gekennzeichnet, dass** ein Dekontaminierungsmittel in einer Mühle gemahlen und anschließend das Probenmaterial, die Probenmaterial-Schmelzmittel-Mischung oder die erstarrte Schmelze in derselben Mühle gemahlen wird.

17. Verfahren gemäß Anspruch 16, **dadurch gekennzeichnet, dass** das Dekontaminierungsmittel eine erste Teilmenge des Probenmaterials, der Probenmaterial-Schmelzmittel-Mischung oder der erstarrten Schmelze umfasst.

18. Verfahren gemäß Anspruch 16 oder 17, **dadurch gekennzeichnet, dass** das Dekontaminierungsmittel ein probenmaterialfremdes Material umfasst.

## Claims

1. A method for analyzing a sample material, wherein the sample material is ground, shaped into a tablet and subsequently analyzed, wherein the analysis is carried out by means of laser emission spectroscopy and wherein the sample material is at least partially mixed with a fluxing agent **characterized in that** the sample material is preground in a first grinding step, and is ground further in a further grinding step after mixing with the fluxing agent.

2. The method as claimed in claim 1, **characterized in that** a laser is applied to a surface of the tablet at a plurality of positions in the scope of the laser emission spectroscopy.

3. The method as claimed in one of the preceding claims, **characterized in that** the sample material or the sample material/fluxing agent mixture is at least partially melted before the analysis.

4. The method as claimed in claim 3, **characterized in that** solidification of the melt is carried out, the solidified melt already having the tablet shape or being converted into the tablet shape.

5. The method as claimed in claim 3 or 4, **characterized in that** the sample material or the sample material/fluxing agent mixture is cooled during the melting.

6. The method as claimed in one of claims 3 to 5, **characterized in that** at least a fraction, intended for the melting, of the sample material or of the sample material/fluxing agent mixture is preheated before the melting and/or supplementarily heated during the melting.

7. The method as claimed in one of claims 4 to 6, **characterized in that** the sample material or the sample material/fluxing agent mixture is melted by means of laser radiation, plasma radiation, high-temperature infrared radiation, microwave radiation and/or electron radiation.

8. The method as claimed in claims 6 and 7, **characterized in that** at least the fraction, intended for the melting, of the sample material or of the sample material/fluxing agent mixture is preheated by means of laser radiation, and the sample material or the sample material/fluxing agent mixture is melted by means of laser radiation of the same laser.

9. The method as claimed in claim 7 or 8, **characterized in that** the sample material or the sample material/fluxing agent mixture is at least partially melted by means of laser radiation, to which end the same laser is used as for carrying out the subsequent laser emission spectroscopy.

10. The method as claimed in one of claims 4 to 9, **characterized in that** the sample material or the sample material/fluxing agent mixture is melted selectively.

11. The method as claimed in claim 4 or one of the claims dependent on claim 4, **characterized in that** the solidification is carried out in such a way that a vitreous component of the tablet is formed.

12. The method as claimed in claim 4 or one of the claims dependent on claim 4, **characterized in that** the sample material or the sample material/fluxing agent mixture is melted and solidified layerwise.

13. The method as claimed in claim 4 or one of the claims dependent on claim 4, **characterized in that** the solidified melt is ground and subsequently converted into the tablet shape.

14. The method as claimed in claim 4 or one of the claims dependent on claim 4, **characterized in that** the melting and/or the carrying out of the solidification of the melt is monitored in real time.

15. The method as claimed in claim 14, **characterized in that** the monitoring is carried out by using imaging sensors or a pyrometer.

16. The method as claimed in one of the preceding claims, **characterized in that** a decontaminant is ground in a mill, and the sample material, the sample material/fluxing agent mixture or the solidified melt is subsequently ground in the same mill.

17. The method as claimed in claim 16, **characterized in that** the decontaminant comprises a first portion of the sample material, of the sample material/fluxing agent mixture or of the solidified melt.

18. The method as claimed in claim 16 or 17, **characterized in that** the decontaminant comprises a material different to the sample material.

## Revendications

1. Procédé d'analyse d'un matériau échantillon, le matériau échantillon étant broyé, mis sous forme de comprimé puis analysé, l'analyse étant effectuée par spectroscopie à émission laser et le matériau échantillon étant au moins partiellement mélangé avec un flux, **caractérisé en ce que** le matériau échantillon est pré-broyé dans une première étape de broyage et, après mélange avec le flux, est encore broyé dans une étape de broyage supplémentaire.

2. Procédé selon la revendication 1, **caractérisé en ce qu'**une surface du comprimé est soumise à un laser en plusieurs points dans le cadre de la spectroscopie à émission laser.

3. Procédé selon l'une des revendications précédentes, **caractérisé en ce que** le matériau échantillon ou le mélange matériau échantillon-flux est au moins partiellement fondu avant l'analyse.

4. Procédé selon la revendication 3, **caractérisé en ce que** la matière fondue est solidifiée, la matière fondue solidifiée ayant déjà la forme d'un comprimé ou étant transformée en comprimé.

5. Procédé selon la revendication 3 ou 4, **caractérisé en ce que** le matériau échantillon ou le mélange matériau échantillon-flux est refroidi lors de la fusion.

6. Procédé selon l'une des revendications 3 à 5, **caractérisé en ce qu'**au moins une partie du matériau échantillon ou du mélange matériau échantillon-flux destiné à la fusion est préchauffée avant la fusion et/ou est soumise à un chauffage supplémentaire pendant la fusion.

7. Procédé selon l'une des revendications 3 à 6, **caractérisé en ce que** le matériau échantillon ou le mélange matériau échantillon-flux est fondu au moyen d'un rayonnement laser, d'un rayonnement plasma, d'un rayonnement infrarouge à haute température, d'un rayonnement micro-ondes et/ou d'un rayonnement d'électrons.

8. Procédé selon les revendications 6 et 7, **caractérisé en ce qu'**au moins la partie du matériau échantillon ou du mélange matériau échantillon-flux prévu destiné à la fusion est préchauffée au moyen d'un rayonnement laser et le matériau échantillon ou le mélange matériau échantillon-flux est fondu au moyen du rayonnement laser du même laser.

9. Procédé selon la revendication 7 ou 8, **caractérisé en ce que** le matériau échantillon ou le mélange matériau échantillon-flux est au moins partiellement fondu au moyen d'un rayonnement laser, le même laser que celui utilisé pour la spectroscopie à émission laser ultérieure étant utilisé pour cela.

10. Procédé selon l'une des revendications 3 à 9, **caractérisé en ce que** le matériau échantillon ou le mélange matériau échantillon-flux est fondu sélectivement.

11. Procédé selon la revendication 4 ou l'une des revendications dépendantes de la revendication 4, **caractérisé en ce que** la solidification est effectuée de manière à former un composant vitreux du comprimé.

12. Procédé selon la revendication 4 ou l'une des revendications dépendantes de la revendication 4, **caractérisé en ce que** le matériau échantillon ou le mélange matériau échantillon-flux est fondu par couches et solidifié.

13. Procédé selon la revendication 4 ou l'une des revendications dépendantes de la revendication 4, **caractérisé en ce que** la matière fondue solidifiée est broyée puis transformée en comprimé.

14. Procédé selon la revendication 4 ou l'une des revendications dépendantes de la revendication 4, **caractérisé en ce que** la fusion et/ou la solidification de la matière fondue sont surveillées en temps réel.

15. Procédé selon la revendication 14, **caractérisé en ce que** la surveillance est effectuée à l'aide de capteurs d'imagerie ou d'un pyromètre.

16. Procédé selon l'une des revendications précédentes, **caractérisé en ce qu'**un agent de décontamination est broyé dans un broyeur puis le matériau échantillon, le mélange matériau échantillon-flux ou la matière fondue solidifiée est broyé (e) dans le même broyeur.

17. Procédé selon la revendication 16, **caractérisé en ce que** l'agent de décontamination comprend une première quantité partielle du matériau échantillon, du mélange matériau échantillon-flux ou de la matière fondue solidifiée.

18. Procédé selon la revendication 16 ou 17, **caractérisé en ce que** l'agent de décontamination comprend un matériau étranger au matériau échantillon.
